# EUROPEAN PATENT APPLICATION

(11) **EP 1 584 614 A1**
(43) Date of publication of application: **12.10.2005**
(21) Application number: 04701092.1
(22) Date of filing: 09.01.2004
(51) Int. Cl.: C07C 211/54, C07C 211/58, H05B 33/14, H05B 33/22

(54) **AROMATIC AMINE DERIVATIVE AND ORGANIC ELECTROLUMINESCENCE ELEMENT**

(30) Priority: 16.01.2003 JP 2003007762
(71) Applicant: IDEMITSU KOSAN CO., LTD., Tokyo 100-8321 (JP)
(72) Inventor: KAWAMURA, Hisayuki, 2990293 (JP)
(74) Representative: Gille Hrabal Struck Neidlein Prop Roos
(86) International application number: PCT/JP2004/000119
(87) International publication number: WO 2004/063142

(57) **Abstract**

A novel aromatic amine derivative having an asymmetric structure; and an organic electroluminescence device comprising a cathode, an anode and an organic thin film layer which is disposed between the cathode and the anode and comprises at least one layer comprising a light emitting layer, wherein at least one layer in the organic thin film layer comprises the above aromatic amine derivative singly or as a component of a mixture. Crystallization of the molecules is suppressed, and the yield in the production of the organic electroluminescence device can be increased.

## Description

### TECHNICAL FIELD

The present invention relates to a novel aromatic amine derivative and an organic electroluminescence element ("electroluminescence" will be occasionally referred to as "EL", and "electroluminescence element" will be occasionally referred to as "EL device", hereinafter) utilizing the derivative. More particularly, the present invention relates to a novel aromatic amine derivative exhibiting suppressed crystallization of the molecules and increasing the yield in the production of the organic EL device and an organic EL device utilizing the derivative.

### BACKGROUND ART

An organic EL device is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported by C. W. Tang of Eastman Kodak Company (C. W. Tang and S. A. Vanslyke, Applied Physics Letters, Volume 51, Pages 913, 1987), many studies have been conducted on organic EL devices using organic materials as the constituting materials. Tang et al. used a laminate structure using tris(8-hydroxyquinolinol)aluminum for the light emitting layer and a triphenyldiamine derivative for the hole transporting layer. Advantages of the laminate structure are that the efficiency of hole injection into the light emitting layer can be increased, that the efficiency of forming excited particles which are formed by blocking and recombining electrons injected from the cathode can be increased, and that excited particles formed within the light emitting layer can be enclosed. As the structure of the organic EL device, a two-layered structure having a hole transporting (injecting) layer and an electron transporting and light emitting layer and a three-layered structure having a hole transporting (injecting) layer, a light emitting layer and an electron transporting (injecting) layer are well known. To increase the efficiency of recombination of injected holes and electrons in the devices of the laminate type, the structure of the device and the process for forming the device have been studied.

In general, when an organic EL device is driven or stored in an environment of a high temperature, adverse effects such as a change in the emitted color, a decrease in the efficiency of light emission, an increase in the voltage for driving and a decrease in the life of light emission arise. To prevent the adverse effects, it has been necessary that the glass transition temperature (Tg) of the hole transporting material be elevated. For this purpose, it is necessary that the hole transporting material have many aromatic groups in the molecule (for example, aromatic diamine derivatives described in the specification of the United States Patent No. 4,720,432; aromatic condensed ring diamine derivatives described in the specification of the United States Patent No. 5,061,569; and tetraphenylbenzidine compounds described in Japanese Patent Application Laid-Open No. Heisei 7(1995)-126615) and, in general, compounds having a structure having 8 to 12 benzene rings have been preferably used.

However, when many aromatic groups are present in the molecule, crystallization tends to take place during the formation of a thin layer using the hole transporting material in the preparation of an organic EL device, and the crystallization causes problems in that the outlet of a crucible used for the vapor deposition is clogged and that defects caused by the crystallization are formed in the thin layer, and the yield in the production of the organic EL device decreases.

### DISCLOSURE OF THE INVENTION

The present invention has been made to overcome the above problems and has an object of providing a novel aromatic amine derivative exhibiting suppressed crystallization of the molecules and increasing the yield in the production of the organic EL device and an organic EL device using the derivative.

As the result of intensive studies by the present inventors to achieve the above object, it was found that the above object could be achieved by using a novel aromatic amine derivative having an asymmetric structure represented by the following general formula (1) as a material for the organic EL device and, in particular, for the hole transporting material. The present invention has been completed based on this knowledge.

The present invention provides an aromatic amine derivative represented by following general formula (1):

A-L-B (1)

wherein A represents a diarylamino group represented by: 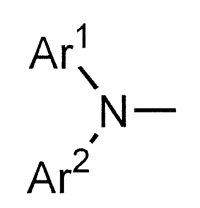 B represents a diarylamino group represented by: 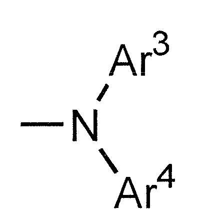 Ar¹ to Ar⁴ each independently representing a substituted or unsubstituted aryl group having 5 to 50 nuclear atoms, and the two diarylamino groups represented by A and B being not same with each other; and
L represents a linking group comprising a substituted or unsubstituted arylene group having 5 to 50 nuclear atoms or a linking group comprising a plurality of substituted or unsubstituted arylene groups having 5 to 50 nuclear atoms bonded with each other through a single bond, oxygen atom, sulfur atom, nitrogen atom or a saturated or unsaturated divalent aliphatic hydrocarbon group having 1 to 20 nuclear carbon atoms.

The present invention also provides an organic EL device comprising a cathode, an anode and an organic thin film layer which is disposed between the cathode and the anode and comprises at least one layer comprising a light emitting layer, wherein at least one layer in the organic thin film layer comprises an aromatic amine derivative described above singly or as a component of a mixture.

### THE MOST PREFERRED EMBODIMENT TO CARRY OUT THE INVENTION

The aromatic amine derivative of the present invention comprises a compound represented by the following the general formula (1):

A-L-B (1)

In general formula (1), the two groups represented by A and B are not the same with each other. In other words, the aromatic amine compound of the present invention has an asymmetric structure.

In general formula (1), A represents a diarylamino group represented by: 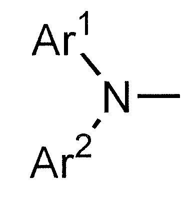 and B represents a diarylamino group represented by: 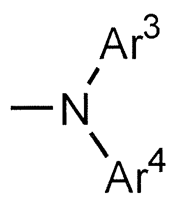 Ar¹ to Ar⁴ each independently represent a substituted or unsubstituted aryl group having 5 to 50 nuclear atoms.

Examples of the aryl group represented by Ar¹ to Ar⁴ include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, fluoranthenyl group, fluorenyl group, 1-pyrrolyl group, 2-pyrrolyl group, 3-pyrrolyl group, pyrazinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 9-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthriinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrohn-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrohn-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methyl-pyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group and 4-t-butyl-3-indolyl group.

Among these groups, phenyl group, naphthyl group, biphenyl group, anthranyl group, phenanthryl group, pyrenyl group, chrysenyl group, fluoranthenyl group and fluorenyl groups are preferable.

In general formula (1), L represents (I) a linking group comprising a substituted or unsubstituted arylene group having 5 to 50 nuclear atoms or (II) a linking group comprising a plurality of substituted or unsubstituted arylene groups having 5 to 50 nuclear atoms bonded with each other through (II-1) the single bond, (II-2) oxygen atom (-O-), (II-3) sulfur atom (-S-), (II-4) nitrogen atom (-NH- or -NR-, R representing a substituent) or (II-5) a saturated or unsaturated divalent aliphatic hydrocarbon group having 1 to 20 nuclear carbon atoms.

Examples of the arylene group having 5 to 50 nuclear atoms in (I) and (II) include 1,4-phenylene group, 1,2-phenylene group, 1,3-phenylene group, 1,4-naphthylene group, 2,6-naphthylene group, 1,5-naphthylene group, 9,10-anthranylene group, 9,10-phenanthrenylene group, 3,6-phenanthrenylene group, 1,6-pyrenylene group, 2,7-pyrenylene group, 6,12-chrysenylene group, 4,4'-biphenylene group, 3,3'-biphenylene group, 2,2'-biphenylene group, 2,7-fluorenylene group, 2,5-thiophenylene group, 2,5-silacyclopentadienylene group and 1,5-oxadiazolylene group. Among these groups, 1,4-phenylene group, 1,2-phenylene group, 1,3-phenylene group, 1,4-naphthylene group, 9,10-anthranylene group, 6,12-chrysenylene group, 4,4'-biphenylene group, 3,3'-biphenylene group, 2,2'-biphenylene group and 2,7-fluorenylene group are preferable.

The saturated or unsaturated divalent aliphatic hydrocarbon group having 1 to 20 nuclear carbon atom of (II-5) may be any of linear, branched and cyclic groups. Examples of the above group include methylene group, ethylene group, propylene group, isopropylene group, ethylidene group, cyclohexylidene group and adamantylene group.

Examples of the substituent to the groups represented by Ar¹ to Ar⁴ and L include substituted and unsubstituted aryl groups having 5 to 50 nuclear atoms, substituted and unsubstituted alkyl groups having 1 to 50 carbon atoms, substituted and unsubstituted alkoxyl groups having 1 to 50 carbon atoms, substituted and unsubstituted aralkyl groups having 1 to 50 carbon atoms, substituted and unsubstituted aryloxyl groups having 5 to 50 nuclear atoms, substituted and unsubstituted arylthio groups having 5 to 50 nuclear atoms, substituted and unsubstituted alkoxycarbonyl groups having 1 to 50 carbon atoms, amino group substituted with a substituted or unsubstituted aryl group having 5 to 50 nuclear atoms, halogen atoms, cyano group, nitro group and hydroxyl group.

Examples of the substituted and unsubstituted aryl groups having 5 to 50 nuclear atoms include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, fluoranthenyl group, fluorenyl group, 1-pyrrolyl group, 2-pyrrolyl group, 3-pyrrolyl group, pyrazinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 9-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrohn-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methyl-pyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group and 4-t-butyl-3-indolyl group.

Examples of the substituted and unsubstituted alkyl groups having 1 to 50 carbon atoms include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxy-isopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triamino-propyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3- tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, cyclopropyl group, cyclobutyl group, cyclopentyl group, cyclohexyl group, 4-methylcyclohexyl group, 1-adamantyl group, 2-adamantyl group, 1-norbornyl group and 2-norbornyl group.

The substituted and unsubstituted alkoxyl groups having 1 to 50 carbon atoms are represented by -OY. Examples of the group represented by Y include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydxoxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3- tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group and 1,2,3-trinitropropyl group.

Examples of the substituted and unsubstituted aralkyl groups having 1 to 50 carbon atoms include benzyl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, 2-β-naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group and 1-chloro-2-phenylisopropyl group.

The substituted and unsubstituted aryloxyl groups having 5 to 50 carbon atoms are represented by -OY'. Examples of the group represented by Y' include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, 2-pyrrolyl group, 3-pyrrolyl group, pyrazinyl group, 2-pyridinyl group, 3-pyridinyl group, 4-pyridinyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 1-phenoxazinyl group, 2-phenoxazinyl group, 3-phenoxazinyl group, 4-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methyl- pyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl- 3-indolyl group and 4-t-butyl-3-indolyl group.

The substituted and unsubstituted arylthio groups having 5 to 50 carbon atoms are represented by -SY". Examples of the group represented by Y" include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, 2-pyrrolyl group, 3-pyrrolyl group, pyrazinyl group, 2-pyridinyl group, 3-pyridinyl group, 4-pyridinyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridrinyl group, 7-phenanthridinyl group, 8-phenanthrichnyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 1-phenoxazinyl group, 2-phenoxazinyl group, 3-phenoxazinyl group, 4-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methyl- pyrrol-5-yl group, 3-methylpyrrol-1-yl group, 3-methyl-pyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl- 3-indolyl group and 4-t-butyl-3-indolyl group.

The substituted and unsubstituted alkoxycarbonyl group having 1 to 50 carbon atoms are represented by -COOZ. Examples of the group represented by Z include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanolsopropyl group, 2,3-dicyano-t-butyl group, 1,2,3- tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group and 1,2,3-trinitropropyl group.

The amino group substituted with a substituted or unsubstituted aryl group having 5 to 50 nuclear atoms is represented by -NPQ. Examples of the groups represented by P and Q include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, 2-pyrrolyl group, 3-pyrrolyl group, pyrazinyl group, 2-pyridinyl group, 3-pyridinyl group, 4-pyridinyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 1-phenoxazinyl group, 2-phenoxazinyl group, 3-phenoxazinyl group, 4-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methyl-pyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl- 3-indolyl group and 4-t-butyl-3-indolyl group. The groups represented by P and Q may be the same with or different from each other.

A plurality of the above substituents may be bonded to each other to form a ring. Examples of the divalent group forming a ring include tetramethylene group, pentamethylene group, hexamethylene group, diphenylmethan-2,2'-diyl group, diphenylethan-3,3'-diyl group and diphenylpropan-4,4'-diyl group.

Examples of the halogen atom include fluorine atom, chlorine atom, bromine atom and iodine atom.

Examples of the aromatic amine derivative represented by general formula (1) are shown in the following. However, the aromatic amine derivative is not limited to the compounds shown as the examples. In the following, Me means methyl group, and iPr means isopropyl group.

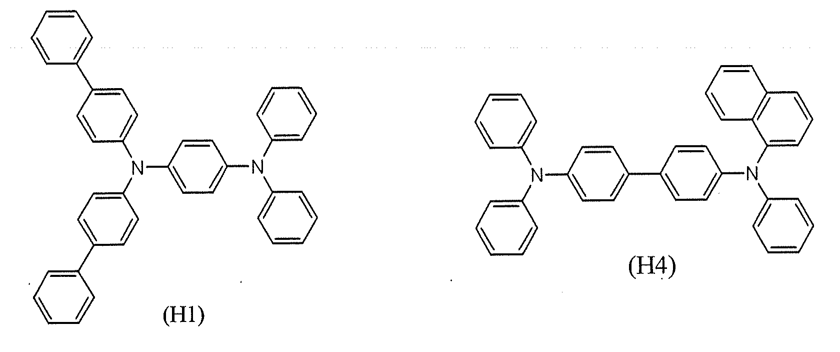

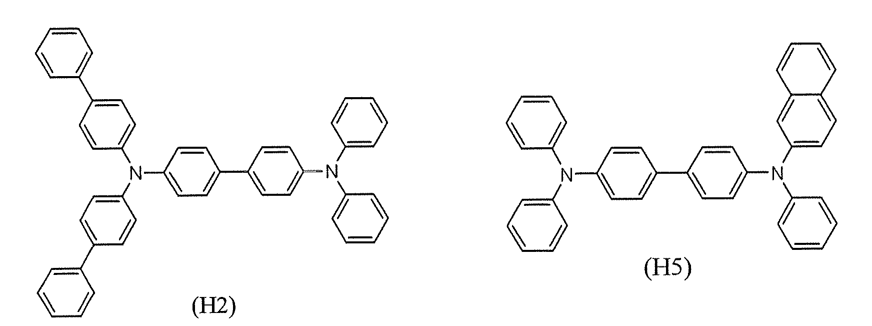

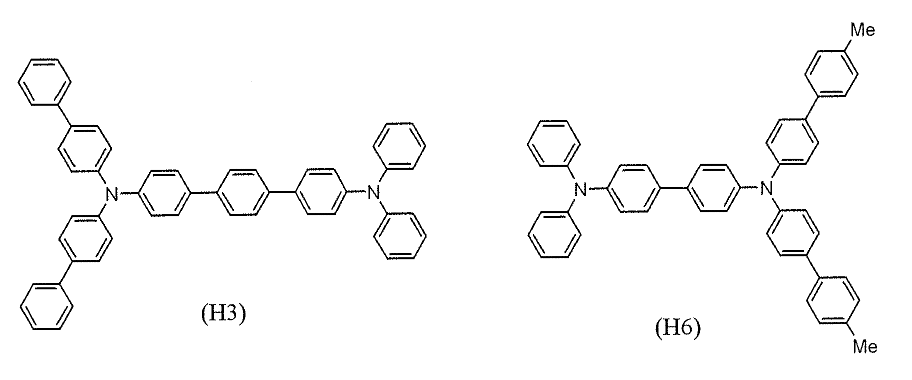

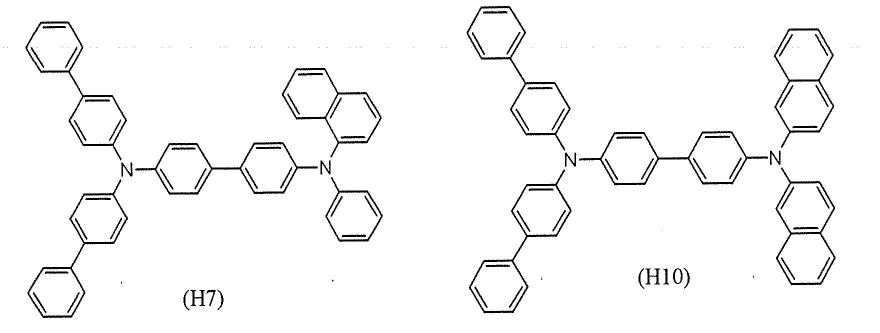

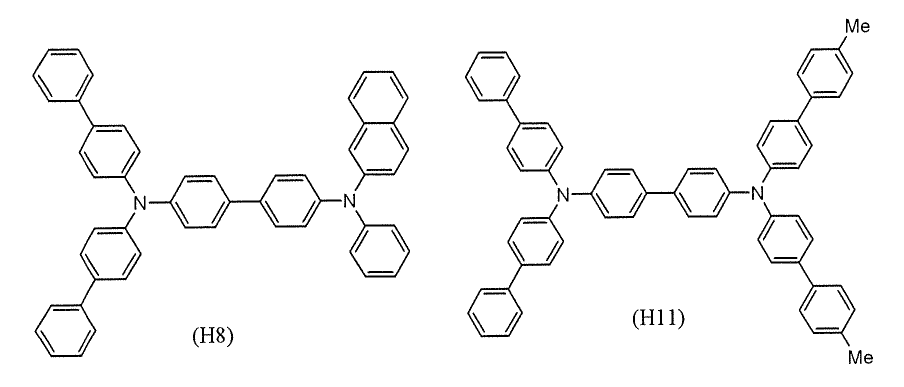

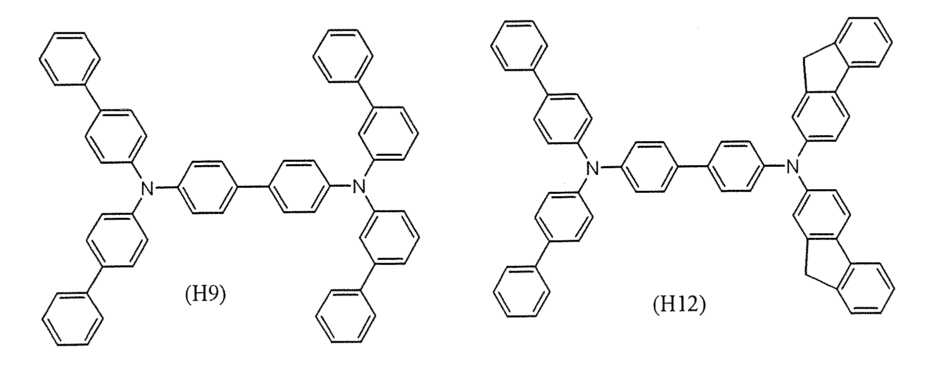

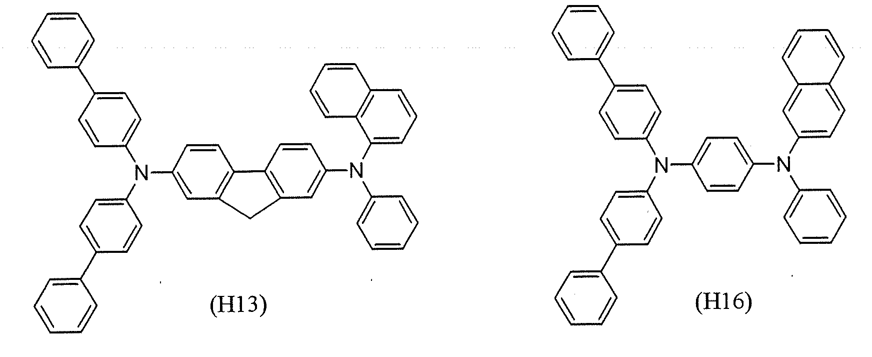

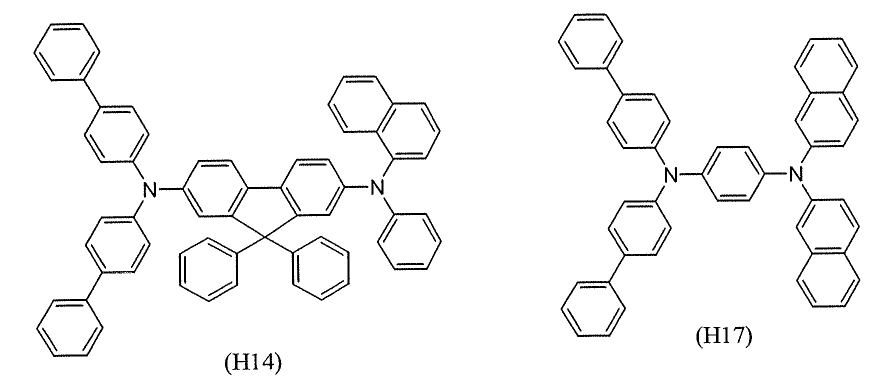

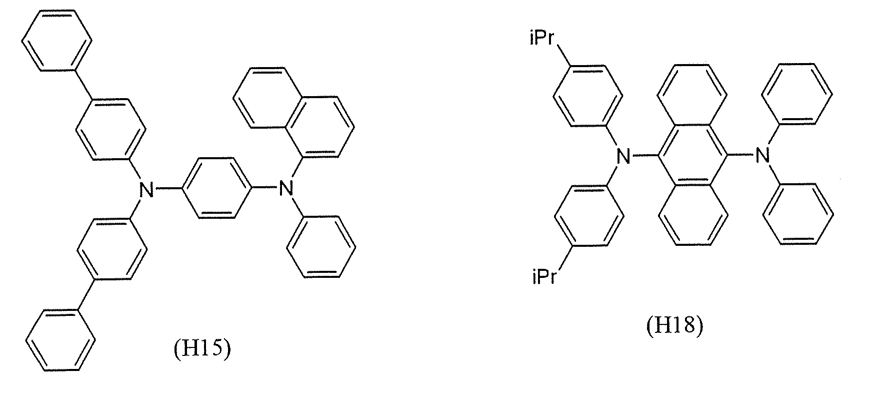

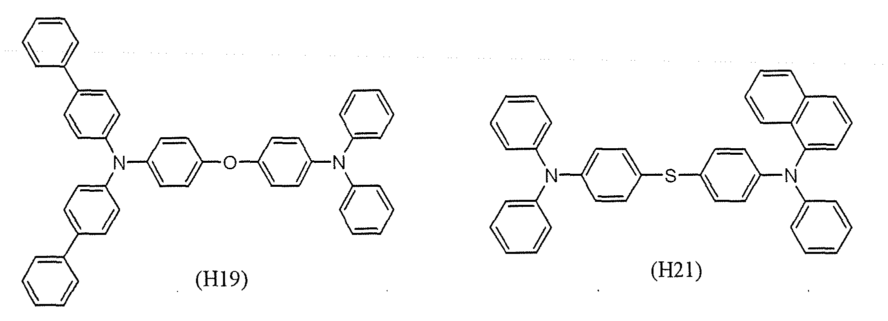

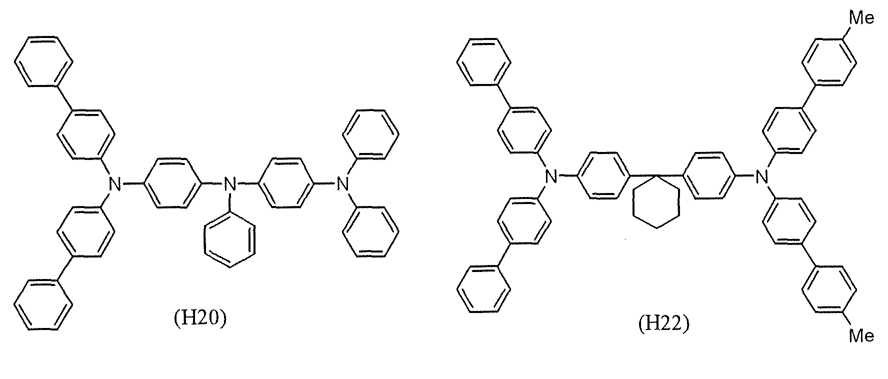

The organic EL device of the present invention will be described in the following.

The organic electroluminescence device of the present invention comprises a cathode, an anode and an organic thin film layer which is disposed between the cathode and the anode and comprises at least one layer comprising the light emitting layer, wherein at least one layer in the organic thin film layer comprises the aromatic amine derivative described above singly or as a component of a mixture.

It is preferable that, in the organic EL device of the present invention, the organic thin film layer comprises a hole transporting zone, and the hole transporting zone comprises the aromatic amine derivative of the present invention singly or as a component of a mixture. It is more preferable that the organic thin film layer comprises a hole transporting layer, and the hole transporting layer comprises the aromatic amine derivative of the present invention singly or as a component of a mixture. It is still more preferable that the hole transporting layer comprises the aromatic amine derivative of the present invention as the main component.

The construction of the organic EL device of the present invention will be described in the following.

### (1) Construction of the organic EL device

Typical examples of the construction of the organic EL device of the present invention include:
[1] An anode / a light emitting layer / a cathode;
[2] An anode / a hole injecting layer / a light emitting layer / a cathode;
[3] An anode / a light emitting layer / an electron injecting layer / a cathode;
[4] An anode / a hole injecting layer / a light emitting layer / an electron injecting layer / a cathode;
[5] An anode / an organic semiconductor layer / a light emitting layer / a cathode;
[6] An anode / an organic semiconductor layer / an electron barrier layer / a light emitting layer / a cathode;
[7] An anode / an organic semiconductor layer / a light emitting layer / an adhesion improving layer / a cathode;
[8] An anode / a hole injecting layer / a hole transporting layer / a light emitting layer / an electron injecting layer / a cathode;
[9] An anode / an insulating layer / a light emitting layer / an insulating layer / a cathode;
[10] An anode / an inorganic semiconductor layer / an insulating layer / a light emitting layer / an insulating layer / a cathode;
[11] An anode / an organic semiconductor layer / an insulating layer / a light emitting layer / an insulating layer / a cathode;
[12] An anode / an insulating layer / a hole injecting layer / a hole transporting layer / a light emitting layer / an insulating layer / a cathode; and
[13] An anode / an insulating layer / a hole injecting layer / a hole transporting layer / a light emitting layer / an electron injecting layer / a cathode.

Among the above constructions, in general, construction [8] is preferable. However, the construction of the organic EL device is not limited to those shown above as the examples.

The aromatic amine derivative of the present invention may be used in any layer in the organic thin film layer of the organic EL device. The aromatic amine derivative can be used in the light emitting zone or the hole transporting zone. It is preferable that the aromatic amine derivative is used in the hole transporting zone and, more preferably, in the hole transporting layer since the crystallization of the molecules can be suppressed, and the yield in the production of the organic EL device can be increased.

It is preferable that the organic thin film layer comprises 30 to 100% by mole of the aromatic amine derivative of the present invention.

### (2) Substrate which transmits light

The organic EL device of the present invention is prepared on a substrate which transmits light. The substrate which transmits light is the substrate which supports the organic EL device. It is preferable that the substrate which transmits light has a transmittance of light of 50% or greater in the visible region of 400 to 700 nm and is flat and smooth.

Examples of the substrate which transmits light include glass plates and synthetic resin plates. Specific examples of the glass plate include plates made of soda-lime glass, glass containing barium and strontium, lead glass, aluminosilicate glass, borosilicate glass, barium borosilicate glass and quartz. Specific examples of the synthetic resin plate include plates made of polycarbonate resins, acrylic resins, polyethylene terephthalate resins, polyether sulfide resins and polysulfone resins.

### (3) Anode

The anode in the organic EL device of the present invention has the function of injecting holes into the hole transporting layer or the light emitting layer. It is effective that the anode has a work function of 4.5 eV or greater. Examples of the material for the anode used in the present invention include indium tin oxide alloys (ITO), tin oxide (NESA), gold, silver, platinum and copper.

The anode can be prepared by forming a thin film of the electrode material described above in accordance with a process such as the vapor deposition process and the sputtering process.

When the light emitted from the light emitting layer is obtained through the anode, it is preferable that the anode has a transmittance of the emitted light greater than 10%. It is also preferable that the sheet resistivity of the anode is several hundred Ω/□ or smaller. The thickness of the anode is, in general, selected in the range of 10 nm to 1 µm and preferably in the range of 10 to 200 nm although the preferable range may be different depending on the used material.

### (4) Light emitting layer

The light emitting layer in the organic EL device has a combination of the following functions:
[1] The injecting function: the function of injecting holes from the anode or the hole injecting layer and injecting electrons from the cathode or the electron injecting layer when an electric field is applied;
[2] The transporting function: the function of transporting injected charges (electrons and holes) by the force of the electric field; and
[3] The light emitting function: the function of providing the field for recombination of electrons and holes and leading the recombination to the emission of light.

The easiness of injection may be different between holes and electrons. The ability of transportation expressed by the mobility may be different between holes and electrons. It is preferable that either one of the charges is transferred.

As the process for forming the light emitting layer, a conventional process such as the vapor deposition process, the spin coating process and the LB process can be used. It is particularly preferable that the light emitting layer is a molecular deposit film. The molecular deposit film is a thin film formed by deposition of a material compound in the gas phase or a thin film formed by solidification of a material compound in a solution or in the liquid phase. In general, the molecular deposit film can be distinguished from the thin film formed in accordance with the LB process (the molecular accumulation film) based on the differences in the aggregation structure and higher order structures and functional differences caused by these structural differences.

As disclosed in Japanese Patent Application Laid-Open No. Showa 57(1982)-51781, the light emitting layer can also be formed by dissolving a binder such as a resin and the material compounds into a solvent to prepare a solution, followed by forming a thin film from the prepared solution in accordance with the spin coating process or the like.

In the present invention, where desired, the light emitting layer may comprise conventional light emitting materials other than the light emitting material comprising the aromatic amine derivative of the present invention, or a light emitting layer comprising other conventional light emitting material may be laminated to the light emitting layer comprising the light emitting material comprising the aromatic amine derivative of the present invention as long as the object of the present invention is not adversely affected.

### (5) Hole injecting and transporting layer (hole transporting zone)

The hole injecting and transporting layer is a layer which helps injection of holes into the light emitting layer and transports the holes to the light emitting region. The layer exhibits a great mobility of holes and, in general, has an ionization energy as small as 5.5 eV or smaller. For the hole injecting and transporting layer, a material which transports holes to the light emitting layer under an electric field of a smaller strength is preferable. A material which exhibits, for example, a mobility of holes of at least 10⁻⁴ cm²/V·sec under application of an electric field of 10⁴ to 10⁶ V/cm is preferable.

When the aromatic amine derivative of the present invention is used in the hole transporting zone, the aromatic amine derivative of the present invention may be used singly or as a mixture with other materials for forming the hole transporting and injecting layer.

The other material which can be used for forming the hole transporting and injecting layer as a mixture with the aromatic amine derivative of the present invention is not particularly limited. The other material can be selected as desired from materials which are conventionally used as the charge transporting material of holes in photoconductive materials and conventional materials which are used for the hole injecting layer in organic EL devices.

Examples include triazole derivatives (United States Patent No. 3,112,197), oxadiazole derivatives (United States Patent No. 3,189,447), imidazole derivatives (Japanese Patent Application Publication No. Showa 37(1962)-16096), polyarylalkane derivatives (United States Patent Nos. 3,615,402, 3,820,989 and 3,542,544; Japanese Patent Application Publication Nos. Showa 45(1970)-555 and Showa 51 (1976)-10983; and Japanese Patent Application Laid-Open Nos. Showa 51(1976)-93224, Showa 55(1980)-17105, Showa 56(1981)-4148, Showa 55(1980)-108667, Showa 55(1980)-156953 and Showa 56(1981)-36656); pyrazoline derivatives and pyrazolone derivatives (United States Patent Nos. 3,180,729 and 4,278,746; and Japanese Patent Application Laid-Open Nos. Showa 55(1980)-88064, Showa 55(1980)-88065, Showa 49(1974)-105537, Showa 55(1980)-51086, Showa 56(1981)-80051, Showa 56(1981)-88141, Showa 57(1982)-45545, Showa 54(1979)-112637 and Showa 55(1980)-74546); phenylenediamine derivatives (United Sates Patent No. 3,615,404; Japanese Patent Application Publication Nos. Showa 51(1976)-10105, Showa 46(1971)-3712 and Showa 47(1972)-25336; and Japanese Patent Application Laid-Open Nos. Showa 54(1979)-53435, Showa 54(1979)-110536 and Showa 54(1979)-119925); arylamine derivatives (United States Patent Nos. 3,567,450, 3,180,703, 3,240,597, 3,658,520, 4,232,103, 4,175,961 and 4,012,376; Japanese Patent Application Publication Nos. Showa 49(1974)-35702 and Showa 39(1964)-27577; Japanese Patent Application Laid-Open Nos. Showa 55(1980)-144250, Showa 56(1981)-119132 and Showa 56(1981)-22437; and West German Patent No. 1,110,518); chalcone derivatives substituted with amino group (United States Patent No. 3,526,501); oxazole derivatives (United States Patent No. 3,257,203); styrylanthracene derivatives (Japanese Patent Application Laid-Open Nos. Showa 56(1981)-46234); fluorenone derivatives (Japanese Patent Application Laid-Open Nos. Showa 54(1979)-110837); hydrazone derivatives (United States Patent No. 3,717,462; and Japanese Patent Application Laid-Open Nos. Showa 54(1979)-59143, Showa 55(1980)-52063, Showa 55(1980)-52064, Showa 55(1980)-46760, Showa 55(1980)-85495, Showa 57(1982)-11350, Showa 57(1982)-148749 and Heisei 2(1990)-311591); stilbene derivatives (Japanese Patent Application Laid-Open Nos. Showa 61(1986)-210363, Showa 61(1986)-228451, Showa 61(1986)-14642, Showa 61(1986)-72255, Showa 62(1987)-47646, Showa 62(1987)-36674, Showa 62(1987)-10652, Showa 62(1987)-30255, Showa 60(1985)-93455, Showa 60(1985)-94462, Showa 60(1985)-174749 and Showa 60(1985)-175052); silazane derivatives (United States Patent No. 4,950,950); polysilane-based compounds (Japanese Patent Application Laid-Open No. Heisei 2(1990)-204996); aniline-based copolymers (Japanese Patent Application Laid-Open No. Heisei 2(1990)-282263); and electrically conductive macromolecular oligomers (in particular, thiophene oligomers) disclosed in Japanese Patent Application Laid-Open No. Heisei 1(1989)-211399.

Besides the above materials which can be used as the material for the hole injecting layer, porphyrin compounds (compounds disclosed in Japanese Patent Application Laid-Open No. Showa 63(1988)-295695); and aromatic tertiary amine compounds and styrylamine compounds (United States Patent No. 4,127,412 and Japanese Patent Application Laid-Open Nos. Showa 53(1978)-27033, Showa 54(1979)-58445, Showa 54(1979)-149634, Showa 54(1979)-64299, Showa 55(1980)-79450. Showa 55(1980)-144250, Showa 56(1981)-119132, Showa 61(1986)-295558, Showa 61(1986)-98353 and Showa 63(1988)-295695) are preferable, and the aromatic tertiary amines are more preferable.

Further examples include compounds having two condensed aromatic rings in the molecule which are described in the United States Patent No. 5,061,569 such as 4,4'-bis(N-(1-naphthyl)-N-phenylamino)-biphenyl (referred to as NPD, hereinafter) and a compound in which three triphenylamine units are bonded together in a star-burst shape, which is described in Japanese Patent Application Laid-Open No. Heisei 4(1992)-308688, such as 4,4',4"-tris(N-(3-methylphenyl)-N-phenylamino)-triphenylamine (referred to as MTDATA, hereinafter).

The aromatic dimethylidene-based compounds described above as the examples of the material for the light emitting layer and inorganic compounds such as Si of the p-type and SiC of the p-type can also be used as the material for the hole injecting layer.

The hole injecting and transporting layer can be formed by preparing a thin film of the aromatic amine derivative of the present invention in accordance with a conventional process such as the vacuum vapor deposition process, the spin coating process, the casting process and the LB process. The thickness of the hole injecting and transporting layer is not particularly limited. In general, the thickness is 5 nm to 5 µm. The hole injecting and transporting layer may comprise a single layer comprising one or more materials described above or may be a laminate comprising a hole injecting and transporting layer comprising materials different from the materials of the hole injecting and transporting layer described above as long as the aromatic amine derivative of the present invention is comprised in the hole injecting and transporting zone.

An organic semiconductor layer may be disposed as a layer helping the injection of holes or electrons into the light emitting layer. As the organic semiconductor layer, a layer having a conductivity of 10⁻¹⁰ S/cm or greater is preferable. As the material for the organic semiconductor layer, oligomers containing thiophene, and conductive oligomers such as oligomers containing arylamine and conductive dendrimers such as dendrimers containing arylamine which are disclosed in Japanese Patent Application Laid-Open No. Heisei 8(1996)-193191, can be used.

### (6) Electron injecting layer

The electron injecting and transporting layer is a layer which helps injection of electrons into the light emitting layer and exhibits a great mobility of electrons. The adhesion improving layer is an electron injecting layer comprising a material exhibiting improved adhesion with the cathode. As the material used for the electron injecting layer, metal complexes of 8-hydroxyquinoline and derivatives thereof are preferable.

Examples of metal complexes of 8-hydroxyquinoline and the derivative thereof include metal chelated oxinoid compounds including chelate compounds of oxines (in general, 8-quinolinol or 8-hydroxyquinoline). For example, tris(8-quinolinol)aluminum (Alq) can be used as the electron injecting material.

Examples of the oxadiazole derivative include electron transfer compounds represented by the following general formulae: 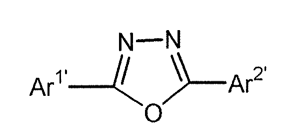 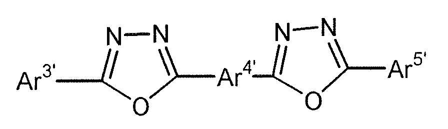 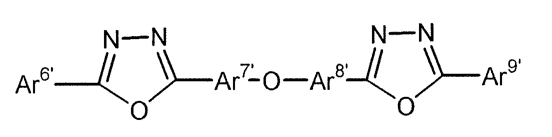 In the above formulae, Ar^{1'}, Ar^{2'}, Ar^{3'}, Ar^{5'}, Ar^{6'} and Ar^{9'} each represent a substituted or unsubstituted aryl group and may represent the same group or different groups. Ar^{4'}, Ar^{7'} and Ar^{8'} each represent a substituted or unsubstituted arylene group and may represent the same group or different groups.

Examples of the aryl group include phenyl group, biphenyl group, anthranyl group, perylenyl group and pyrenyl group. Examples of the arylene group include phenylene group, naphthylene group, biphenylene group, anthranylene group, perylenylene group and pyrenylene group. Examples of the substituent include alkyl groups having 1 to 10 carbon atoms, alkoxyl groups having 1 to 10 carbon atoms and cyano group. As the electron transfer compound, compounds which can form thin films are preferable.

Specific examples of the electron transfer compound include the following compounds:

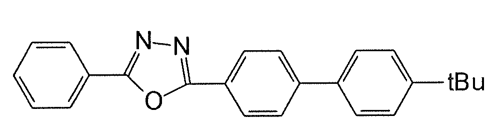

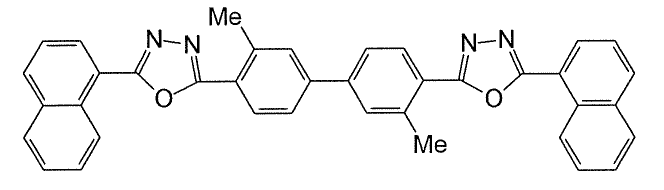

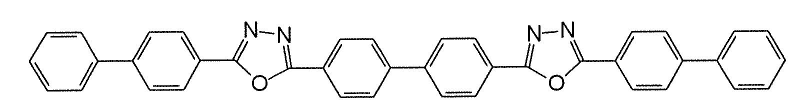

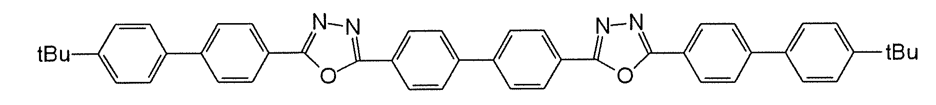

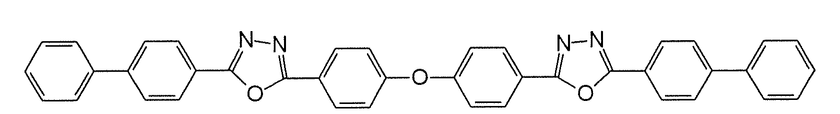

The organic EL device of the present invention may comprise a reducing dopant in the region of electron transport or in the interfacial region of the cathode and the organic thin film layer. The reducing dopant is defined as a substance which can reduce a compound having the electron transporting property. Various compounds can be used as the reducing dopant as long as the compounds have a prescribed reductive property. For example, at least one substance selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, organic complexes of alkali metals, organic complexes of alkaline earth metals and organic complexes of rare earth metals can be advantageously used.

Preferable examples of the reducing dopant include substances having a work function of 2.9 eV or smaller, specific examples of which include at least one alkali metal selected from the group consisting of Na (the work function: 2.36 eV), K (the work function: 2.28 eV), Rb (the work function: 2.16 eV) and Cs (the work function: 1.95 eV) and at least one alkaline earth metal selected from the group consisting of Ca (the work function: 2.9 eV), Sr (the work function: 2.0 to 2.5 eV) and Ba (the work function: 2.52 eV). Among the above substances, at least one alkali metal selected from the group consisting of K, Rb and Cs is more preferable, Rb and Cs are still more preferable, and Cs is most preferable as the reducing dopant. Alkali metals have great reducing ability, and the luminance of the emitted light and the life of the organic EL device can be increased by addition of a relatively small amount of the alkali metal into the electron injecting zone. As the reducing dopant having a work function of 2.9 eV or smaller, combinations of two or more alkali metals are also preferable. Combinations having Cs such as the combinations of Cs and Na, Cs and K, Cs and Rb and Cs, Na and K are more preferable. The reducing ability can be efficiently exhibited by the combination having Cs. The luminance of emitted light and the life of the organic EL device can be increased by adding the combination having Cs into the electron injecting zone.

The organic EL device of the present invention may further comprise an electron injecting layer which is constituted with an insulating material or a semiconductor and disposed between the cathode and the organic layer. By the electron injecting layer, leak of electric current can be effectively prevented, and the electron injecting property can be improved. As the insulating material, at least one metal compound selected from the group consisting of alkali metal chalcogenides, alkaline earth metal chalcogenides, alkali metal halides and alkaline earth metal halides is preferable. It is preferable that the electron injecting layer is constituted with the above substance such as the alkali metal chalcogenide since the electron injecting property can be further improved. Preferable examples of the alkali metal chalcogenide include Li₂O, LiO, Na₂S, Na₂Se and NaO. Preferable examples of the alkaline earth metal chalcogenide include CaO, BaO, SrO, BeO, BaS and CaSe. Preferable examples of the alkali metal halide include LiF, NaF, KF, LiCl, KCl and NaCl. Preferable examples of the alkaline earth metal halide include fluoride such as CaF₂, BaF₂, SrF₂, MgF₂ and BeF₂ and halides other than the fluorides.

Examples of the semiconductor constituting the electron transporting layer include oxides, nitrides and oxide nitrides of at least one metal selected from Ba, Ca, Sr, Yb, Al, Ga, In, Li, Na, Cd, Mg, Si, Ta, Sb and Zn used singly or in combination of two or more. It is preferable that the inorganic compound constituting the electron transporting layer forms a crystallite or amorphous insulating thin film. When the electron injecting layer is constituted with the insulating thin film described above, a more uniform thin film can be formed, and defects of pixels such as dark spots can be decreased. Examples of the inorganic compound include alkali metal chalcogenides, alkaline earth metal chalcogenides, alkali metal halides and alkaline earth metal halides which are described above.

### (7) Cathode

For the cathode, a material such as a metal, an alloy, a conductive compound or a mixture of these materials which has a small work function (4 eV or smaller) is used as the electrode material so that electrons are injected into the electron transporting layer or the light emitting layer. Examples of the electrode material include sodium, sodium-potassium alloys, magnesium, lithium, magnesium-silver alloys, aluminum/aluminum oxide, aluminum-lithium alloys, indium and rare earth metals.

The cathode can be prepared by forming a thin film of the electrode material described above in accordance with a process such as the vapor deposition process and the sputtering process.

When the light emitted from the light emitting layer is obtained through the cathode, it is preferable that the cathode has a transmittance of the emitted light greater than 10 %.

It is also preferable that the sheet resistivity of the cathode is several hundred Ω/□ or smaller. The thickness of the cathode is, in general, selected in the range of 10 nm to 1 µm and preferably in the range of 50 to 200 nm.

### (8) Insulating layer

Defects in pixels tend to be formed in organic EL device due to leak and short circuit since an electric field is applied to ultra-thin films. To prevent the formation of the defects, a layer of a thin film having an insulating property may be inserted between the pair of electrodes.

Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide and vanadium oxide. Mixtures and laminates of the above compounds can also be used.

### (9) Process for producing the organic EL device

To prepare the organic EL device of the present invention, for example, the anode, the light emitting layer and, where necessary, the hole injecting and transporting layer and the electron injecting and transporting layer are formed in accordance with the above process using the above materials, and the cathode is formed in the last step. The organic EL device may be prepared by forming the above layers in the order reverse to that described above, i.e., the cathode being formed in the first step and the anode in the last step.

An embodiment of the process for preparing an organic EL device having a construction in which an anode, a hole injecting layer, a light emitting layer, an electron injecting layer and a cathode are disposed successively on a substrate transmitting light will be described in the following.

On a suitable substrate which transmits light, a thin film made of a material for the anode is formed in accordance with the vapor deposition process or the sputtering process so that the thickness of the formed thin film is 1 µm or smaller and preferably in the range of 10 to 200 nm. The formed thin film is used as the anode. Then, a hole injecting layer is formed on the anode. The hole injecting layer can be formed in accordance with the vacuum vapor deposition process, the spin coating process, the casting process or the LB process, as described above. The vacuum vapor deposition process is preferable since a uniform film can be easily obtained and the possibility of formation of pin holes is small. When the hole injecting layer is formed in accordance with the vacuum vapor deposition process, in general, it is preferable that the conditions are suitably selected in the following ranges: the temperature of the source of the deposition: 50 to 450°C; the vacuum: 10⁻⁷ to 10⁻³ Torr; the rate of deposition: 0.01 to 50 nm/second; the temperature of the substrate: -50 to 300°C and the thickness of the film: 5 nm to 5 µm; although the conditions of the vacuum vapor deposition are different depending on the used compound (the material for the hole injecting layer) and the crystal structure and the recombination structure of the hole injecting layer to be formed.

Then, the light emitting layer is formed on the hole injecting layer formed above. Using a desired organic light emitting material, a thin film of the organic light emitting material can be formed in accordance with the vacuum vapor deposition process, the sputtering process, the spin coating process or the casting process, and the formed thin film is used as the light emitting layer. The vacuum vapor deposition process is preferable since a uniform film can be easily obtained and the possibility of formation of pin holes is small. When the light emitting layer is formed in accordance with the vacuum vapor deposition process, in general, the conditions of the vacuum vapor deposition process can be selected in the same ranges as those described for the vacuum vapor deposition of the hole injecting layer although the conditions are different depending on the used compound.

An electron injecting layer is formed on the light emitting layer formed above. Similarly to the hole injecting layer and the light emitting layer, it is preferable that the electron injecting layer is formed in accordance with the vacuum vapor deposition process since a uniform film must be obtained. The conditions of the vacuum vapor deposition can be selected in the same ranges as those described for the vacuum vapor deposition of the hole injecting layer and the light emitting layer.

When the vapor deposition process is used, the aromatic amine derivative of the present invention can be vapor deposited in combination with other materials although the situation may be different depending on which layer in the light emitting zone or in the hole transporting zone comprises the aromatic amine derivative. When the spin coating process is used, the aromatic amine derivative can be incorporated into the formed layer by using a mixture of the aromatic amine derivative with other materials.

A cathode is formed on the electron injecting layer formed above in the last step, and an organic EL device can be obtained.

The cathode is made of a metal and can be formed in accordance with the vacuum vapor deposition process or the sputtering process. It is preferable that the vacuum vapor deposition process is used in order to prevent formation of damages on the lower organic layers during the formation of the film.

In the above preparation of the organic EL device, it is preferable that the above layers from the anode to the cathode are formed successively while the preparation system is kept in a vacuum after being evacuated once.

The process for forming the layers in the organic EL device of the present invention is not particularly limited. A conventional process such as the vacuum vapor deposition process and the spin coating process can be used. The organic thin film layer which is used in the organic EL device of the present invention and comprises the compound represented by general formula (1) described above can be formed in accordance with a conventional process such as the vacuum vapor deposition process and the molecular beam epitaxy process (the MBE process) or, using a solution prepared by dissolving the compounds into a solvent, in accordance with a coating process such as the dipping process, the spin coating process, the casting process, the bar coating process and the roll coating process.

The thickness of each layer in the organic thin film layer in the organic EL device of the present invention is not particularly limited. In general, an excessively thin layer tends to have defects such as pin holes, and an excessively thick layer requires a high applied voltage to decrease the efficiency. Therefore, a thickness in the range of several nanometers to 1 µm is preferable.

The organic EL device which can be prepared as described above emits light when a direct voltage of 5 to 40 V is applied in the condition that the anode is connected to a positive electrode (+) and the cathode is connected to a negative electrode (-). When the connection is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be used.

The present invention will be described more specifically with reference to examples in the following. However, the present invention is not limited to the examples.

### Example 1 (Synthesis of N,N-diphenyl-1-amino-N,N-dibiphenylyl-4-aminobenzene (H1))

### (1) Synthesis of di-4-biphenylylamine

Into a 100 ml three-necked flask, 10.0 g of 4-bromobiphenyl (manufactured by TOKYO KASEI KOGYO Co., Ltd.), 4.32 g of sodium t-butoxide (manufactured by WAKO Pure Chemcal Industries, Ltd.) and 42 mg of palladium acetate (manufactured by WAKO Pure Chemcal Industries, Ltd.) were placed. After a stirrer rod was placed into the flask containing the above substances, rubber caps were fitted to the two openings at the sides, and a Dimroth condenser for refluxing was fitted to the opening at the center. A three-way stopcock and a balloon containing argon gas were successively attached to the top of the Dimroth condenser. The system was purged with the argon gas in the balloon three times using a vacuum pump.

Then, 60 ml of dehydrated toluene (manufactured by WAKO Pure Chemcal Industries, Ltd.), 2.04 ml of benzylamine (manufactured by TOKYO KASEI KOGYO Co., Ltd.) and 169 µl of tris-t-butylphosphine (manufactured by ALDRICH Company; a 2.22 mole/liter toluene solution) were added through the rubber septum using a syringe, and the resultant mixture was stirred at the room temperature for 5 minutes.

The flask was placed in an oil bath, and the temperature was raised slowly to 120°C while the solution was stirred. After 7 hours, the flask was removed out of the oil bath to terminate the reaction and left standing for 12 hours under the atmosphere of argon.

The reaction fluid was transferred to a separation funnel, and precipitates were dissolved by adding 300 ml of dichloromethane. The obtained solution was washed with 60 ml of a saturated aqueous solution of sodium chloride, and the organic layer was dried with anhydrous potassium carbonate. Potassium carbonate was removed by filtration, and the solvent was removed from the resultant organic layer by distillation. To the obtained residue, 200 ml of toluene and 40 ml of ethanol were added and, after a drying tube was attached, the residue was completely dissolved by heating at 80°C. The obtained mixture was left standing for 12 hours to slowly cool to the room temperature and, thus, the recrystallization was conducted.

The formed crystals were separated by filtration and dried in vacuo at 60°C, and 6.73 g of N,N-di(4-biphenylyl)benzylamine was obtained.

Into a 300 ml single-necked flask, 1.35 g of N,N-di(4-biphenylyl)-benzylamine and 135 mg of palladium-active carbon (manufactured by WAKO Pure Chemcal Industries, Ltd.; the content of palladium: 10% by weight) were placed, and 100 ml of chloroform and 20 ml of ethanol were added to dissolve the above substances.

After a stirrer rod was placed into the flask, a three-way stopcock attached with a balloon filled with 2 liters of hydrogen gas was attached to the flask, and the system inside the flask was purged with the hydrogen gas 10 times using a vacuum pump. The consumed hydrogen gas was replenished with fresh hydrogen gas, and the volume of the hydrogen gas was adjusted at 2 liters. The reaction fluid was vigorously stirred at the room temperature. After the stirring for 30 hours, 100 ml of dichloromethane was added, and the catalyst was removed by filtration.

The obtained fluid was transferred to a separation funnel and washed with 50 ml of a saturated aqueous solution of potassium hydrogencarbonate. The organic layer was separated and dried with anhydrous potassium carbonate. After filtration, the solvent in the organic layer was removed by distillation, and 50 ml of toluene was added to the obtained residue for recrystallization. The formed crystals were separated by filtration and dried in vacuo at 50°C, and 0.99 g of di-4-biphenylylamine was obtained.

### (2) Synthesis of 4-bromo-N,N-dibiphenylylaniline

Under a stream of argon, 10 g of di-4-biphenylylamine obtained above in (1), 7.3 g of 1,4-dibromobenzene (manufactured by TOKYO KASEI KOGYO Co., Ltd.), 3 g of sodium t-butoxide (manufactured by HIROSHIMA WAKO Co., Ltd.), 0.5 g of bis(triphenylphosphine)-palladium(II) chloride (manufactured by TOKYO KASEI KOGYO Co., Ltd.) and 500 ml of xylene were placed into a reactor, and the reaction was allowed to proceed at 130°C for 24 hours.

After the reaction mixture was cooled, 1,000 ml of water was added, and the resultant mixture was filtered through a Celite filter. The filtrate was treated by extraction with toluene and dried with anhydrous magnesium sulfate. The resultant solution was concentrated under a reduced pressure, and the obtained crude product was purified in accordance with the column chromatography and recrystallized from toluene. The formed crystals were separated by filtration and dried, and 8.1 g of 4-bromo-N,N-dibiphenylylaniline was obtained.

### (3) Synthesis of Compound (H1)

Under a stream of argon, 2 g of 4-bromo-N,N-dibiphenylylaniline obtained above in (2), 1 g of N,N-diphenylamine (manufactured by TOKYO KASEI KOGYO Co., Ltd.), 0.5 g of sodium t-butoxide (manufactured by HIROSHIMA WAKO Co., Ltd.), 0.1 g of bis(triphenylphosphine)- palladium(II) chloride (manufactured by TOKYO KASEI KOGYO Co., Ltd.) and 300 ml of xylene were placed into a reactor, and the reaction was allowed to proceed at 130°C for 24 hours.

After the reaction mixture was cooled, 500 ml of water was added, and the resultant mixture was filtered through a Celite filter. The filtrate was treated by extraction with toluene and dried with anhydrous magnesium sulfate. The resultant solution was concentrated under a reduced pressure, and the obtained crude product was purified in accordance with the column chromatography and recrystallized from toluene. The formed crystals were separated by filtration and dried, and 1.6 g of a light yellow powder was obtained.

The obtained powder was analyzed in accordance with the field desorption mass spectroscopy (FD-MS) analysis and identified to be N,N-diphenyl-1-amino-N,N-dibiphenylyl-4-aminobenzene (H1) since the main peak was found at m/z=564, which corresponded to C₄₂H₃₂N₂=564.

### Example 2 (Synthesis of N,N-diphenyl-4-amino-N',N'-dibiphenylyl-4'-amino-1,1'-biphenyl (H2))

### (1) Synthesis of 4'-bromo-N,N- dibiphenyl-4-amino- 1,1'-biphenyl

Under a stream of argon, 10 g of di-4-biphenylylamine obtained in Example 1 (1), 9.7 g of 4,4'-dibromobiphenyl (manufactured by TOKYO KASEI KOGYO Co., Ltd.), 3 g of sodium t-butoxide (manufactured by HIROSHIMA WAKO Co., Ltd.), 0.5 g of bis(triphenylphosphine)palladium(II) chloride (manufactured by TOKYO KASEI KOGYO Co., Ltd.) and 500 ml of xylene were placed into a reactor, and the reaction was allowed to proceed at 130°C for 24 hours.

After the reaction mixture was cooled, 1,000 ml of water was added, and the resultant mixture was filtered through a Celite filter. The filtrate was treated by extraction with toluene and dried with anhydrous magnesium sulfate. The resultant solution was concentrated under a reduced pressure, and the obtained crude product was purified in accordance with the column chromatography and recrystallized from toluene. The formed crystals were separated by filtration and dried, and 9.1 g of 4'-bromo-N,N-dibiphenylyl-4-amino-1,1'-biphenyl was obtained.

### (2) Synthesis of Compound (H2)

Under a stream of argon, 2.3 g of 4'-bromo-N,N-dibiphenylyl-4-amino-1,1'-biphenyl obtained above in (1), 1 g of N,N-diphenylamine (manufactured by TOKYO KASEI KOGYO Co., Ltd.), 0.5 g of sodium t-butoxide (manufactured by HIROSHIMA WAKO Co., Ltd.), 0.1 g of bis(triphenylphosphine)palladium(II) chloride (manufactured by TOKYO KASEI KOGYO Co., Ltd.) and 300 ml of xylene were placed into a reactor, and the reaction was allowed to proceed at 130°C for 24 hours.

After the reaction mixture was cooled, 500 ml of water was added, and the resultant mixture was filtered through a Celite filter. The filtrate was treated by extraction with toluene and dried with anhydrous magnesium sulfate. The resultant solution was concentrated under a reduced pressure, and the obtained crude product was purified in accordance with the column chromatography and recrystallized from toluene. The formed crystals were separated by filtration and dried, and 1.6 g of a light yellow powder was obtained.

The obtained powder was analyzed in accordance with the FD-MS analysis and identified to be N,N-diphenyl-4-amino-N',N'-dibiphenylyl-4'-amino-1,1'-biphenyl (H2) since the main peak was found at m/z=640, which corresponded to C₄₈H₃₆N₂=640.

### Example 3 (Synthesis of N,N-diphenyl-4-amino-N",N"-dibiphenylyl-4"-amino-p-terphenyl (H3))

### (1) Synthesis of 4"-bromo-N,N-dibiphenylyl-4-amino-p-terphenyl

Under a stream of argon, 10 g of di-4-biphenylylamine (manufactured by TOKYO KASEI KOGYO Co., Ltd.), 12.1 g of 4,4"-dibromo-p- terphenyl (manufactured by LANCASTER Company), 3 g of sodium t-butoxide (manufactured by HIROSHIMA WAKO Co., Ltd.), 0.5 g of bis(triphenylphosphine)palladium(II) chloride (manufactured by TOKYO KASEI KOGYO Co., Ltd.) and 500 ml of xylene were placed into a reactor, and the reaction was allowed to proceed at 130°C for 24 hours.

After the reaction mixture was cooled, 1,000 ml of water was added, and the resultant mixture was filtered through a Celite filter. The filtrate was treated by extraction with toluene and dried with anhydrous magnesium sulfate. The resultant solution was concentrated under a reduced pressure, and the obtained crude product was purified in accordance with the column chromatography and recrystallized from toluene. The formed crystals were separated by filtration and dried, and 9.4 g of 4"-bromo-N,N-dibiphenylyl-4-amino-p-terphenyl was obtained.

### (2) Synthesis of Compound (H3)

Under a stream of argon, 2.6 g of 4"-bromo-N,N-dibiphenylyl-4-amino-p-terphenyl obtained above in (1), 1 g of N,N-diphenylamine (manufactured by TOKYO KASEI KOGYO Co., Ltd.), 0.5 g of sodium t-butoxide (manufactured by HIROSHIMA WAKO Co., Ltd.), 0.1 g of bis(triphenylphosphine)palladium(II) chloride (manufactured by TOKYO KASEI KOGYO Co., Ltd.) and 300 ml of xylene were placed into a reactor, and the reaction was allowed to proceed at 130°C for 24 hours.

After the reaction mixture was cooled, 500 ml of water was added, and the resultant mixture was filtered through a Celite filter. The filtrate was treated by extraction with toluene and dried with anhydrous magnesium sulfate. The resultant solution was concentrated under a reduced pressure, and the obtained crude product was purified in accordance with the column chromatography and recrystallized from toluene. The formed crystals were separated by filtration and dried, and 1.7 g of a light yellow powder obtained.

The obtained powder was analyzed in accordance with the FD-MS analysis and identified to be N,N-diphenyl-4-amino-N",N"-dibiphenylyl-4"-amino-p-terphenyl (H3) since the main peak was found at m/z=716, which corresponded to C₅₄H₄₀N₂=716.

### Example 4 (Synthesis of N,N,N'-triphenyl-4-amino-N'-(1-naphthyl)-amino-1,1'-biphenyl (H4))

Under a stream of argon, 2.3 g of 4'-bromo-N,N-dibiphenylyl-4-amino-1,1'-biphenyl obtained in Example 2 (1), 1 g of N-phenyl-1-naphthylamine (manufactured by TOKYO KASEI KOGYO Co., Ltd.), 0.5 g of sodium t-butoxide (manufactured by HIROSHIMA WAKO Co., Ltd.), 0.1 g of bis(triphenylphosphine)palladium(II) chloride (manufactured by TOKYO KASEI KOGYO Co., Ltd.) and 300 ml of xylene were placed into a reactor, and the reaction was allowed to proceed at 130°C for 24 hours.

After the reaction mixture was cooled, 500 ml of water was added, and the resultant mixture was filtered through a Celite filter. The filtrate was treated by extraction with toluene and dried with anhydrous magnesium sulfate. The resultant solution was concentrated under a reduced pressure, and the obtained crude product was purified in accordance with the column chromatography and recrystallized from toluene. The formed crystals were separated by filtration and dried, and 2.0 g of a light yellow powder obtained.

The obtained powder was analyzed in accordance with the FD-MS analysis and identified to be N,N,N'-triphenyl-4-amino-N'-(1-naphthyl)-amino-1,1'-biphenyl (H4) since the main peak was found at m/z=690, which corresponded to C₅₂H₃₈N₂=690.

### Example 5 (Synthesis of N,N,N'-triphenyl-4-amino-N'-(2-naphthyl)-amino-1,1'-biphenyl (H5))

Under a stream of argon, 2.3 g of 4'-bromo-N,N-dibiphenylyl-4-amino-1,1'-biphenyl obtained in Example 2 (1), 1 g of N-phenyl-2-naphthylamine (manufactured by TOKYO KASEI KOGYO Co., Ltd.), 0.5 g of sodium t-butoxide (manufactured by HIROSHIMA WAKO Co., Ltd.), 0.1 g of bis(triphenylphosphine)palladium(II) chloride (manufactured by TOKYO KASEI KOGYO Co., Ltd.) and 300 ml of xylene were placed into a reactor, and the reaction was allowed to proceed at 130°C for 24 hours.

After the reaction mixture was cooled, 500 ml of water was added, and the resultant mixture was filtered through a Celite filter. The filtrate was treated by extraction with toluene and dried with anhydrous magnesium sulfate. The resultant solution was concentrated under a reduced pressure, and the obtained crude product was purified in accordance with the column chromatography and recrystallized from toluene. The formed crystals were separated by filtration and dried, and 1.9 g of a light yellow powder obtained.

The obtained powder was analyzed in accordance with the FD-MS analysis and identified to be N,N,N'-triphenyl-4-amino-N'-(2-naphthyl)-amino-1,1'-biphenyl (H5) since the main peak was found at m/z=690, which corresponded to C₅₂H₃₈N₂=690.

### Example 6 (Synthesis of N,N-diphenyl-4-amino-N',N'-di(4'-methyl-4-biphenylyl)-4'-amino-1,1'-biphenyl (H6))

### (1) Synthesis of 4-iodo-4'-methylbiphenyl

Into a reactor, 75 g of 4-methylbiphenyl (manufactured by TOKYO KASEI KOGYO Co., Ltd.), 19.2 g of orthoperiodic acid (manufactured by WAKO Pure Chemcal Industries, Ltd.), 64.3 g of iodine, 230 g of acetic acid and 7.6 ml of concentrated sulfuric acid were placed, and the reaction was allowed to proceed at 70°C for 2 hours.

After the reaction was completed, the reaction mixture was cooled to the room temperature and injected into 850 ml of methanol under stirring. The formed crystals were separated by filtration and then recrystallized from 2.1 liters of acetonitrile, and 73 g of 4-iodo-4'-methylbiphenyl was obtained.

### (2) Synthesis of di(4'-methyl-4-biphenylyl)amine

Into a 100 ml three-necked flask, 10.0 g of 4-iodo-4'-methylbiphenyl obtained above in (1), 4.32 g of sodium t-butoxide (manufactured by WAKO Pure Chemcal Industries, Ltd.) and 42 mg of palladium acetate (manufactured by WAKO Pure Chemcal Industries, Ltd.) were placed. After a stirrer rod was placed into the flask, rubber caps were fitted to the two openings at the sides, and a Dimroth condenser for refluxing was fitted to the opening at the center. A three-way stopcock and a balloon containing argon gas were successively attached to the top of the Dimroth condenser. The system was purged with argon gas in the balloon three times using a vacuum pump.

Then, 60 ml of dehydrated toluene (manufactured by WAKO Pure Chemcal Industries, Ltd.), 2.04 ml of benzylamine (manufactured by TOKYO KASEI KOGYO Co., Ltd.) and 169 µl of tris-t-butylphosphine (manufactured by ALDRICH Company; a 2.22 mole/liter toluene solution) were added through the rubber septum using a syringe, and the resultant mixture was stirred at the room temperature for 5 minutes.

The flask was placed in an oil bath, and the temperature was raised slowly to 120°C while the solution was stirred. After 7 hours, the flask was removed out of the oil bath to terminate the reaction and left standing for 12 hours under the atmosphere of argon.

The reaction fluid was transferred to a separation funnel, and precipitates were dissolved by adding 300 ml of dichloromethane. The obtained solution was washed with 60 ml of a saturated aqueous solution of sodium chloride, and the organic layer was dried with anhydrous potassium carbonate. Potassium carbonate was removed by filtration, and the solvent was removed from the resultant organic layer by distillation. To the obtained residue, 200 ml of toluene and 40 ml of ethanol were added. After a drying tube was attached to the flask, the residue was completely dissolved by heating at 80°C. The obtained mixture was left standing for 12 hours so that the mixture was slowly cooled to the room temperature and the recrystallization took place.

The formed crystals were separated by filtration and dried in vacuo at 60°C, and 6.12 g of N,N-di(4'-methyl-4-biphenylyl)benzylamine was obtained.

Into a 300 ml single-necked flask, 1.35 g of N,N-di-(4'-methyl-4-biphenylyl)benzylamine and 135 mg of palladium-active carbon (manufactured by WAKO Pure Chemcal Industries, Ltd.; the content of palladium: 10% by weight) were placed, and 100 ml of chloroform and 20 ml of ethanol were added to dissolve the above compounds.

After a stirrer rod was placed into the flask, a three-way stopcock attached with a balloon filled with 2 liters of hydrogen gas was attached to the flask, and the system inside the flask was purge with the hydrogen gas 10 times using a vacuum pump. The consumed hydrogen gas was replenished with fresh hydrogen gas, and the volume of the hydrogen gas was adjusted at 2 liters. The reaction fluid was vigorously stirred at the room temperature. After the stirring for 30 hours, 100 ml of dichloromethane was added, and the catalyst was removed by filtration.

The obtained fluid was transferred to a separation funnel and washed with 50 ml of a saturated aqueous solution of potassium hydrogencarbonate. The organic layer was separated and dried with anhydrous potassium carbonate. After filtration, the solvent in the organic layer was removed by distillation, and 50 ml of toluene was added to the obtained residue for recrystallization. The formed crystals were separated by filtration and dried in vacuo at 50°C, and 0.83 g of di(4'-methyl-4-biphenylyl)amine was obtained.

### (3) Synthesis of Compound (H6)

Under a stream of argon, 2.3 g of 4'-bromo-N,N-dibiphenylyl-4-amino-1,1'-biphenyl obtained in Example 2 (1), 1.8 g of di-(4'-methyl-4-biphenylyl)amine obtained above in (2), 0.5 g of sodium t-butoxide (manufactured by HIROSHIMA WAKO Co., Ltd.), 0.1 g of bis(triphenylphosphine)palladium(II) chloride (manufactured by TOKYO KASEI KOGYO Co., Ltd.) and 300 ml of xylene were placed into a reactor, and the reaction was allowed to proceed at 130°C for 24 hours.

After the reaction mixture was cooled, 500 ml of water was added, and the resultant mixture was filtered through a Celite filter. The filtrate was treated by extraction with toluene and dried with anhydrous magnesium sulfate. The resultant solution was concentrated under a reduced pressure, and the obtained crude product was purified in accordance with the column chromatography and recrystallized from toluene. The formed crystals were separated by filtration and dried, and 2.3 g of a light yellow powder was obtained.

The obtained powder was analyzed in accordance with the FD-MS analysis and identified to be N,N-diphenyl-4-amino-N',N'-di(4'-methyl-4-biphenylyl)-4'-amino-1,1'-biphenyl (H6) since the main peak was found at m/z=821, which corresponded to C₆₂H₄₈N₂=820.

### Example 7

A glass substrate (manufactured by GEOMATEC Company) of 25 mm × 75 mm × 1.1 mm thickness having an ITO transparent electrode was cleaned by application of ultrasonic wave in isopropyl alcohol for 5 minutes and then by exposure to ozone generated by ultraviolet light for 30 minutes.

The cleaned glass substrate having the transparent electrode was attached to a substrate holder of a vacuum vapor deposition apparatus. On the surface of the cleaned substrate at the side having the transparent electrode, a film of N,N'-bis(N,N'-diphenyl-4-aminophenyl)-N,N-diphenyl-4,4'-diamino-1,1'-biphenyl (referred to as "TPD232 film", hereinafter) having a thickness of 60 nm was formed in a manner such that the formed film covered the transparent electrode. The formed TPD232 film worked as the hole injecting layer. On the formed TPD232 film, a film of Compound (H1) synthesized above having a thickness of 20 nm was formed. The formed film of Compound (H1) worked as the hole transporting layer. On the formed film of Compound (H1), Compound EM1 shown below was vapor deposited to form a film having a thickness of 40 nm. At the same time, an amine compound having styryl group (D1) shown below was vapor deposited in an amount such that the ratio of the amounts by weight of EM1 to D1 were 40:2. The formed film worked as the light emitting layer. On the formed film, a film of Alq shown below having a thickness of 10 nm was formed. This film worked as the electron injecting layer. On the film formed above, Li (the source of lithium: manufactured by SAES GETTERS Company) as the reducing dopant and Alq were binary vapor deposited, and an Alq:Li film (the thickness: 10 nm) was formed as the electron injecting layer (or the cathode). On the formed Alq:Li film, metallic aluminum was vapor deposited to form a metal cathode, and an organic EL device was prepared.

The efficiency of light emission of the obtained device was measured at a current density of 1 mA/cm². The result is shown in Table 1.

Compound (H1) was highly amorphous, and clogging of the opening for the source of vapor deposition with crystals did not take place during the vapor deposition.

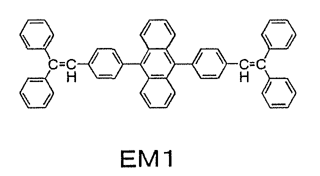

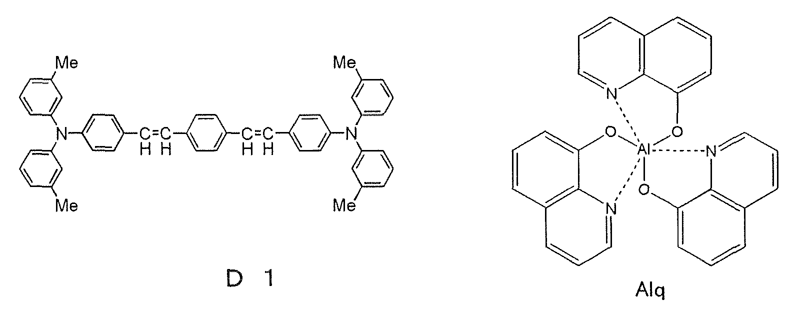

### Example 8

An organic EL device was prepared in accordance with the same procedures as those conducted in Example 7 except that Compound (H2) was used in place of Compound (H1).

The efficiency of light emission of the obtained device was measured at a current density of 1 mA/cm². The result is shown in Table 1.

Compound (H2) was highly amorphous, and clogging of the opening for the source of vapor deposition with crystals did not take place during the vapor deposition.

### Example 9

An organic EL device was prepared in accordance with the same procedures as those conducted in Example 7 except that Compound (H3) was used in place of Compound (H1).

The efficiency of light emission of the obtained device was measured at a current density of 1 mA/cm². The result is shown in Table 1.

Compound (H3) was highly amorphous, and clogging of the opening for the source of vapor deposition with crystals did not take place during the vapor deposition.

### Example 10

An organic EL device was prepared in accordance with the same procedures as those conducted in Example 7 except that Compound (H4) was used in place of Compound (H1).

The efficiency of light emission of the obtained device was measured at a current density of 1 mA/cm². The result is shown in Table 1.

Compound (H4) was highly amorphous, and clogging of the opening for the source of vapor deposition with crystals did not take place during the vapor deposition.

### Example 11

An organic EL device was prepared in accordance with the same procedures as those conducted in Example 7 except that Compound (H5) was used in place of Compound (H1).

The efficiency of light emission of the obtained device was measured at a current density of 1 mA/cm². The result is shown in Table 1.

Compound (H5) was highly amorphous, and clogging of the opening for the source of vapor deposition with crystals did not take place during the vapor deposition.

### Example 12

An organic EL device was prepared in accordance with the same procedures as those conducted in Example 7 except that Compound (H6) was used in place of Compound (H1).

The efficiency of light emission of the obtained device was measured at a current density of 1 mA/cm². The result is shown in Table 1.

Compound (H6) was highly amorphous, and clogging of the opening for the source of vapor deposition with crystals did not take place during the vapor deposition.

### Comparative Example 1

An organic EL device was prepared in accordance with the same procedures as those conducted in Example 7 except that N,N,N',N'-tetra(4-biphenyl)diaminobiphenylene (TBDB) shown below was used in place of Compound (H1).

The efficiency of light emission of the obtained device was measured at a current density of 1 mA/cm². The result is shown in Table 1.

TBDB was highly crystalline. TBDB crystallized at the opening for the source of vapor deposition during the vapor deposition, and the continuous formation of the films could not be conducted.

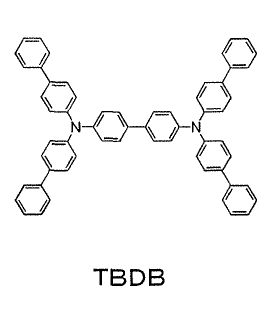

**Table 1**

| | Hole transporting material | Efficiency of light emission (cd/A) | Color of emitted light |
|---|---|---|---|
| Example 7 | H1 | 11.0 | blue |
| Example 8 | H2 | 12.0 | blue |
| Example 9 | H3 | 11.2 | blue |
| Example 10 | H4 | 12.2 | blue |
| Example 11 | H5 | 12.1 | blue |
| Example 12 | H6 | 12.3 | blue |
| Comparative Example 1 | TBDB | 11.5 | blue |

As clearly shown by the above results, when the aromatic amine derivative of the present invention was used as the hole transporting material of the organic EL device, the light emission could be achieved at the same efficiency of light emission as that obtained by using a conventional material, and the films in the device could be formed continuously without clogging of the opening for the source of vapor deposition during the vapor deposition. Therefore, the aromatic amine derivative was very effective for improving the yield in the production of the organic EL device.

### INDUSTRIAL APPLICABILITY

As specifically described in the above, in the aromatic amine derivative of the present invention and the organic EL device utilizing the derivative, crystallization of the molecules is suppressed while the great efficiency of light emission is maintained, and the yield in the production of the organic EL device can be increased.

## Claims

1. An aromatic amine derivative represented by following general formula (1):
A-L-B (1)
wherein A represents a diarylamino group represented by: 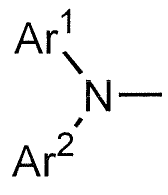 B represents a diarylamino group represented by: 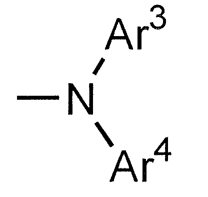 Ar¹ to Ar⁴ each independently representing a substituted or unsubstituted aryl group having 5 to 50 nuclear atoms, and the two diarylamino groups represented by A and B being not same with each other; and
L represents a linking group comprising a substituted or unsubstituted arylene group having 5 to 50 nuclear atoms or a linking group comprising a plurality of substituted or unsubstituted arylene groups having 5 to 50 nuclear atoms bonded with each other through a single bond, oxygen atom, sulfur atom, nitrogen atom or a saturated or unsaturated divalent aliphatic hydrocarbon group having 1 to 20 nuclear carbon atoms.

2. An organic electroluminescence device comprising a cathode, an anode and an organic thin film layer which is disposed between the cathode and the anode and comprises at least one layer comprising a light emitting layer, wherein at least one layer in the organic thin film layer comprises an aromatic amine derivative described in Claim 1 singly or as a component of a mixture.

3. An organic electroluminescence device according to Claim 2, wherein the organic thin film layer comprises a hole transporting zone, and the hole transporting zone comprises an aromatic amine derivative described in Claim 1 singly or as a component of a mixture.

4. An organic electroluminescence device according to Claim 2, wherein the organic thin film layer comprises a hole transporting layer, and the hole transporting layer comprises the aromatic amine derivative singly or as a component of a mixture.

5. An organic electroluminescence device according to Claim 4, wherein the hole transporting layer comprises the aromatic amine derivative as a main component.

6. An organic electroluminescence device according to Claim 2, wherein the organic thin film layer comprises 30 to 100% by mole of the aromatic amine derivative.
